# EUROPÄISCHE PATENTSCHRIFT

(11) **EP 2 337 591 B1**
(45) Veröffentlichungstag und Bekanntmachung des Hinweises auf die Patenterteilung: **22.05.2013**
(21) Anmeldenummer: 09736144.8
(22) Anmeldetag: 07.10.2009
(51) Int. Cl.: A61M 1/02, A61M 5/168, A61M 39/08, A61M 39/22, A61J 1/10, B21D 41/00

(54) **VORRICHTUNG ZUM ÖFFNEN EINER LEITUNG**
DEVICE FOR OPENING A LINE
DISPOSITIF POUR OUVRIR UNE LIGNE

(30) Priorität: 10.10.2008 DE 102008051163
(43) Veröffentlichungstag der Anmeldung: 29.06.2011
(73) Patentinhaber: Fresenius Kabi Deutschland GmbH, 61352 Bad Homburg (DE)
(72) Erfinder: MEISBERGER, Artur, 66606 St. Wendel (DE); MICHEL, Lars, 61191 Rosbach v.d. Höhe (DE)
(74) Vertreter: Brandt, Maximilian
(86) Internationale Anmeldenummer: PCT/EP2009/007203
(87) Internationale Veröffentlichungsnummer: WO 2010/040521

(56) Entgegenhaltungen:
- DE-A1-102005 019 855
- US-A- 3 117 615
- US-A- 3 266 287

## Beschreibung

Die vorliegende Erfindung betrifft eine Vorrichtung zum Öffnen einer Leitung, insbesondere der Leitung eines Blutschlauch- oder Blutbeutelsystems, wobei die Leitung ein den Leitungsdurchlass absperrendes Verschlusselement aufweist, wobei die Vorrichtung wenigstens eine Öffnungseinrichtung aufweist, die derart ausgeführt ist, dass sie eine von außen auf die Leitung wirkende Kraft ausübt, wodurch das Verschlusselement zusammengepresst und die Leitung geöffnet wird.

Eine derartige Vorrichtung zum Öffnen einer Leitung ist beispielsweise aus der DE 10 2005 019 855 A1 bekannt. Die dort offenbarte Öffnungseinrichtung besteht aus zwei Backen, die maschinell oder manuell zusammengeführt werden. Dabei wird der zwischen den Backen befindliche Leitungsabschnitt zusammengedrückt und das Verschlusselement plastisch verformt, so dass ein freier Strömungsquerschnitt in der Leitung entsteht und auch nach Einwirkung der Öffnungseinrichtung frei bleibt.

Das Klinikpersonal und das Personal in Blutbanken hat häufig eine vergleichsweise große Anzahl von Blutbeutelsets zu verarbeiten, um Blut in einem Separator in seine Bestandteile zu trennen und diese Teile in getrennten Behältern aufzubewahren. Damit sind vergleichsweise hohe Anforderungen an das Personal verbunden, da insbesondere das andauernde Öffnen von gängigen Sterilverschlüssen mit einem hohen Kraftaufwand verbunden ist und auf die Dauer zu Schmerzen beim Personal führen kann. Zudem steht das Personal oft unter hohem Zeitdruck, so dass die einzelnen Verfahrensschritte in Zeitspannen von wenigen Sekunden bewerkstelligt werden müssen.

Schließlich besteht die Gefahr, dass Fehlbedienungen auftreten und dass die Sterilverschlüsse versehentlich nicht oder nur teilweise geöffnet werden oder scharfkantige Stellen aufweisen. Insbesondere beim Vorliegenden scharfkantiger Stellen besteht die Gefahr, dass die Blutkomponenten durch Hämolyse Schaden nehmen können.

Es besteht daher die Notwendigkeit, die Verfahrensabläufe zu vereinfachen und zu beschleunigen und dabei gleichzeitig hohe Sicherheits- und Qualitätsstandards zu erfüllen. Dies gilt insbesondere für das Öffnen von Sterilverschlüssen, wie sie aus der DE 10 2005 019 855 A1 bekannt sind.

Wichtig ist, dass die Öffnungseinrichtung zum Öffnen des Verschlußelementes in kürzester Zeit und mit bester Genauigkeit arbeitet. Dabei sollte insbesondere in dem Fall, in dem die Vorrichtung als per Hand betätigte Vorrichtung ausgeführt ist, eine schnelle und einfache Bedienung gewährleistet sein, so dass eine hohe Akzeptanz durch die Nutzer erreicht wird.

Aus der WO 2004/058046 A2 ist ein Blutverarbeitungsgerät bekannt, an dem sich eine Einrichtung zum Öffnen von Sterilverschlüssen in Blutschläuchen befindet. Diese Öffnungseinrichtung ist axial bewegbar und zusätzlich rotierbar. Sie dient zum Öffnen eines Sterilverschlusses mit Brechkonus. Wie oben ausgeführt, sind derartige Sterilverschlüsse in sofern nachteilig, da unter Umständen scharfe Kanten und Bruchstücke des Sterilverschlusses entstehen können, die zu einer Beschädigung der Blutkomponenten führen können. Ein weiterer Nachteil der in der WO 2004/058046 A2 beschriebenen Anordnung besteht darin, dass der beschriebene Mechanismus vergleichsweise aufwendig ist, da ein ständiges Hin- und Herbewegen des Aktuators bewirkt werden muss. Dies ist zudem auch mit einem gewissen Zeitaufwand verbunden.

Aus der EP 1 294 061 B1 ist ein batteriebetriebenes, hydraulisch arbeitendes Pressgerät für den Handbetrieb, das vergleichsweise aufwendig konstruiert ist. Derartige Konstruktionen sind vergleichweise schwer und nicht in schneller Handhabung zu verwenden, wie dies erforderlich ist, wenn es darum geht, mit hoher Präzision und Geschwindigkeit Sterilverschlüsse in Blutbeutel- oder Blutschlauchsystemen zu öffnen.

Entsprechendes gilt für das in der WO 03/084719 A2 offenbarte elektrohydraulische Handwerkzeug, das zur Verpressung von rohrartigen Gegenständen und Kabelschuhen dient. Die offenbarte Konstruktion ist vergleichsweise aufwendig und schwer und für das Öffnen von Sterilverschlüssen daher ungeeignet.

Aus der EP 0 106 182 B1 ist schließlich ein Werkzeug zum Verpressen von Hülsen, Kabelschuhen oder dergleichen bekannt. Auch dieses Werkzeug arbeitet auf hydraulischer Basis. Es ist als Handwerkzeug zur Öffnung von Sterilverschlüssen ungeeignet.

Der vorliegenden Erfindung liegt die Aufgabe zugrunde, eine Vorrichtung der eingangs genannten Art dahingehend weiterzubilden, dass diese in kurzer Zeit und mit hoher Genauigkeit das Öffnen von Sterilverschlüssen ermöglicht, wie sie aus der DE 10 2005 019 855 A1 bekannt sind.

Diese Aufgabe wird durch eine Vorrichtung mit den Merkmalen des Anspruchs 1 gelöst.

Danach ist vorgesehen, dass die Öffnungseinrichtung derart konturiert ist, dass die Leitung nur derart in die Öffnungseinrichtung einlegbar ist, dass die auf die Leitung wirkende Kraft ausschließlich oder zumindest auch in dem Bereich ausgeübt wird, in dem sich das Verschlusselement befindet. Die Leitung bzw. der das Verschlußelement aufweisende Leitungsabschnitt kann somit nur derart in die Öffnungseinrichtung eingelegt werden, dass die von außen auf die Leitung wirkende Presskraft an einer vorbestimmten Stelle wirkt und die Leitung dadurch geöffnet wird. Zwischen der Öffnungseinrichtung und dem fraglichen Leitungsabschnitt besteht somit im Sinne eines Schlüssel-Schloß-Prinzips ein Wirkzusammenhang, durch den verhindert wird, dass versehentlich ein Leitungsabschnitt in die Öffnungseinrichtung eingelegt wird, in dem sich das Verschlußelement nicht befindet. Auf diese Weise kann zuverlässig verhindert werden, dass die Öffnungseinrichtung zwar betätigt wird, dies jedoch nicht zu einem Öffnen der Leitung führt.

Das korrekte Einbringen des Leitungsabschnittes bzw. der Leitung in die Öffnungseinrichtung wird somit durch das genannte Schlüssel-Schloß-Prinzip unterstützt. In einer bevorzugten Ausgestaltung der Erfindung sind des weiteren Mittel vorgesehen, die ein nur teilweises Einlegen der Leitung verhindern. Der Nutzer der Vorrichtung kann somit dahingehend unterstüzt werden, dass verhindert wird, dass die Öffnungseinrichtung betätigt wird, obwohl die Leitung nur teilweise in die Öffnungseinrichtung eingelegt ist. Beispielsweise können diese Mittel über einen Federmechanismus verfügen, der derart ausgestaltet ist, dass die Leitung entweder aus der Öffnungseinrichtung herausgedrückt wird oder in die korrekte Lage in der Öffnungseinrichtung hinein gedrückt wird. Dieser Federmechanismus drückt nicht korrekt plazierte Leitungen bzw. Leitungsabschnitte somit entweder wieder aus der Öffnungseinrichtung bzw. aus deren Aufnahme heraus oder drückt sie in die korrekte Lage, in der ein Öffnen des Verschlußelementes möglich ist.

Vorgesehen sein kann, dass die Öffnungseinrichtung derart ausgeführt ist, dass im Fehlerfall, wenn die Leitung falsch plaziert wurde und der Öffnungsvorgang trotzdem initiiert wurde, das Verschlußelement nicht so stark beschädigt wird, dass die Sterilität der Produkte gefährdet ist. Der Öffnungsvorgang kann somit beispielsweise rechtzeitig abgebrochen werden bzw. das Verschlußelement nur so geringfügig verformt werden, dass die Leitung verschlossen bleibt und damit die Sterilität der Blutprodukte gewährleistet bleibt.

In weiterer Ausgestaltung der Erfindung ist vorgesehen, dass die Vorrichtung oder die Öffnungseinrichtung wenigstens einen Sensor aufweist, der derart ausgeführt ist, dass der die Anwesenheit der Leitung in der Öffnungseinrichtung und/oder deren Position in der Öffnungseinrichtung und/oder wenigstens einen für den Öffnungsvorgang des Verschlußelementes charakteristischen Parameter erfaßt. Wie ausgeführt, kann es sich bei diesem wenigstens einen charakteristischen Parameter um die Kraft, das Drehmoment oder den Motorstrom oder dergleichen handeln, die beim Öffnen des Verschlußelements auftreten.

Die vorliegende Erfindung kann in Form eines Handwerkzeuges ausgeführt sein, das heißt in Form einer handgehaltenen Einrichtung. Vorzugsweise findet diese handegehaltene Einrichtung in Verfahren Anwendung, in denen die Handhabung von Blutbeutelsystemen zur Zellseparation oder zur sonstigen Blutbehandlung zumindest teilweise manuell abläuft. Die Erfindung ist jedoch darauf nicht beschränkt, sondern umfasst z. B. auch Blutbearbeitungsmaschinen, die durch die erfindungsgemäße Vorrichtung gebildet werden oder deren Bestandteil die erfindungsgemäße Vorrichtung darstellt. Bei diesen Blutverarbeitungsmaschinen kann vorgesehen sein, dass weitgehend automatische Prozesse zur Zellseparation oder sonstigen Blutbehandlung verwendet werden. Vorzugsweise ist vorgesehen, dass das Öffnen des Verschlußelementes ebenfalls automatisiert erfolgt bzw. zu einem bestimmten Zeitpunkt innerhalb des Prozesses initiiert wird.

Handelt es sich bei dem Werkzeug um eine handgehaltene Einrichtung, kann vorgesehen sein, dass diese mit der Prozessoreinheit der Vorrichtung über eine drahtgebundene Verbindung oder auch schnurlos in einer Kommunikationsverbindung steht. Diese Kommunikationsverbindung kann beispielsweise dazu dienen, an der handgehaltenen Vorrichtung Informationen anzuzeigen, wie beispielsweise eine Nutzerführung, die dem Nutzer anzeigt, welches Verschlußelement er zu welchem Zeitpunkt mittels der handgehaltenen Einrichtung zu Öffnen hat. In diesem Fall ist das Handwerkzeug besonders einfach und schnell zu bedienen, da dem Nutzer beispielsweise auf einem Display oder akustisch oder auf sonstige Weise angezeigt wird, dass er das Verschlußelement bzw. welches Verschlußelement er zu öffnen hat.

Die genannte Prozessoreinheit kann beispielsweise in einer Blutverarbeitungsmaschine angeordnet sein und der oder die Sensoren in der handgehaltenen Einrichtung.

Wie oben ausgeführt, ist die Erfindung jedoch nicht auf handgehaltene Einrichtungen beschränkt, sondern umfasst ebenfalls den Fall, dass die wenigstens eine Öffnungseinrichtung Bestandteil einer größeren Einheit, insbesondere einer Blutverarbeitungsmaschine ist.

Wie ausgeführt, kann es sich bei der Vorrichtung um ein Blutbehandlungsgerät, insbesondere um ein Gerät zur Zellseparation handeln. Die Erfindung ist jedoch darauf nicht beschränkt.

Der Begriff "Leitung" ist weit zu verstehen und umfasst jeden beliebigen durchströmbaren Bereich, wie beispielsweise Leitungsabschnitte, Schläuche sowie Konnektorbereiche z. B. von Blutbeutelsystemen.

Weitere Einzelheiten und Vorteile der Erfindung werden anhand eines in der Zeichnung dargestellten Ausführungsbeispiels näher erläutert. Es zeigen:
- Figur 1:: eine perspektivische Darstellung eines Teils einer Blutbehandlungseinrichtung mit Öffnungseinrichtung,
- Figur 2:: eine Öffnungseinrichtung in perspektivischer Darstellung in zusammengesetztem Zustand sowie in einer Explosionsdarstellung,
- Figur 3, 4:: eine handgehaltene Vorrichtung zum Öffnen von Sterilverschlüssen in einer perspektivischen Darstellung sowie im geschnittenen Zustand,
- Figur 5, 6:: Detaildarstellungen der Kulissenführung sowie des Rotationskörpers der Einrichtung gemäß Figur 1, 2,
- Figur 7 - 12:: Detaildarstellungen des Rotationskörpers sowie der Kulissenführung mit Öffnungselement,
- Figur 13 - 18:: unterschiedliche Ansichten sowie Konturdarstellungen der Kulissenführung der Einrichtung gemäß Figur 3, 4.

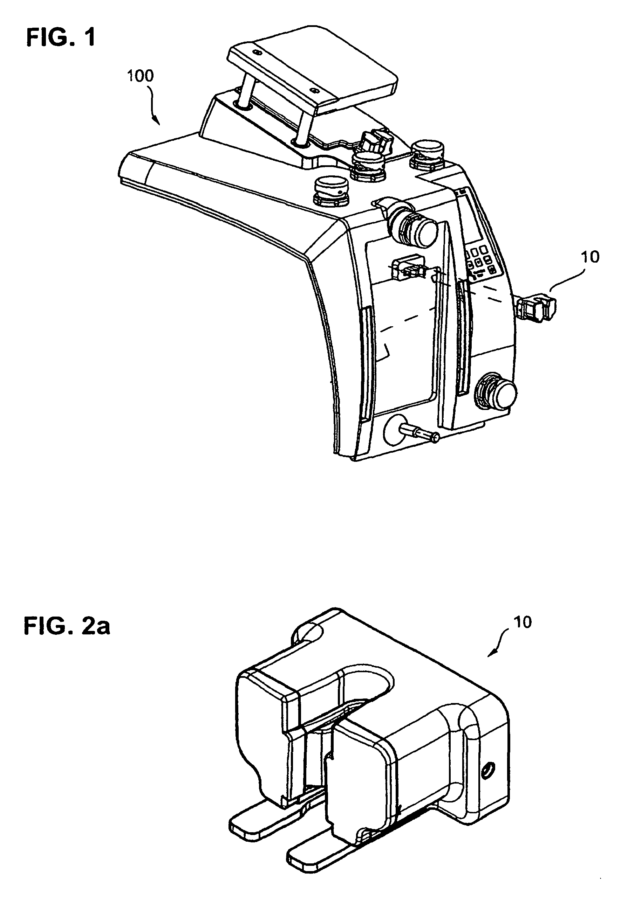

In Figur 1 ist in perspektivischer Darstellung ein Teil 100 eines Blutbehandlungsgerätes zur Zellseparation dargestellt. Dieses Gerät weist an seiner Frontseite eine Öffnungseinrichtung 10 auf, die zum Öffnen eines Verschlußelementes dient, wie es im Detail in der DE 10 2005 019 855 A1 beschrieben ist. Auf diese Druckschrift wird insoweit Bezug genommen.

Die Öffnungseinrichtung 10 wird durch eine nicht näher dargestellte Prozessoreinheit des Gerätes zu bestimmten Zeitpunkten aktiviert, um das Zusammenpressen des Verschlußelementes und damit das Öffnen der Leitung zu bewirken.

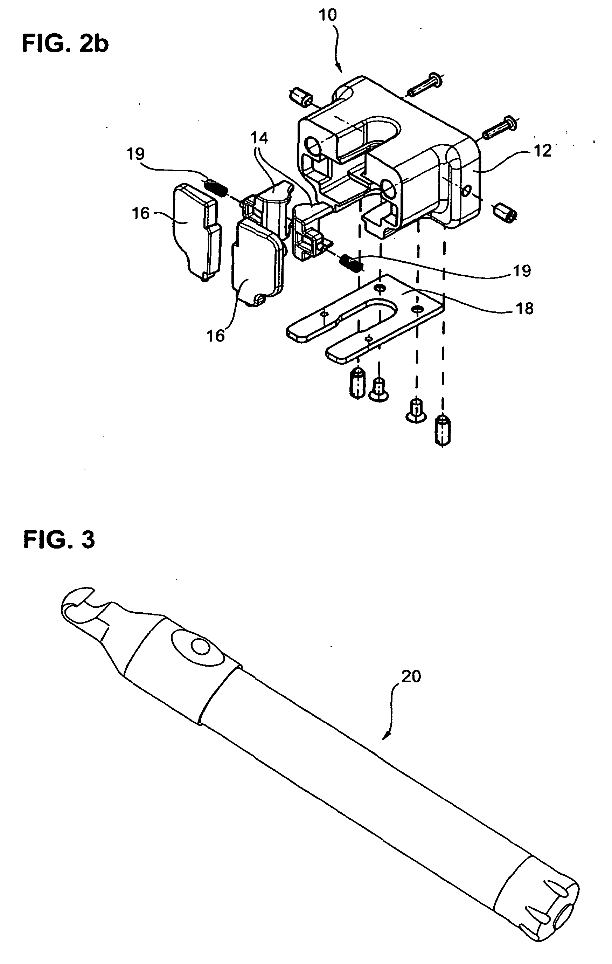

Figur 2a zeigt in vergrößerter perspektivischer Darstellung die Öffnungseinrichtung 10, Figur 2b zeigt die Anordnung in einer Explosionsdarstellung. Mit dem Bezugszeichen 12 ist das Gehäuse der Anordnung gekennzeichnet. Das Bezugszeichen 14 kennzeichnet die beiden zangenartig zusammenwirkenden Elemente eines Mechanismus, der verhindert, dass der Schlauchabschnitt nur teilweise in die dargestellte Öffnungseinrichtung 10 eingelegt wird. Wie dies aus Figur 2b hervorgeht, sind die beiden Backen 14 durch Federn 19 belastet. Diese federbelasteten Backen 14 bewirken, dass ein Schlauch entweder vollständig in die Öffnungseinrichtung eingedrückt wird oder wieder ausgedrückt wird, sofern er nur teilweise eingelegt wurde. Die eigentlichen vorzugsweise zangenartig zusammenwirkenden Backen oder Zangen zum Öffnen des Verschlusselementes befinden sich aus Sicht des Betrachters hinter dem durch die Backen 14 begrenzten Bereich und sind in den Figuren nicht dargestellt.

Das korrekte Einbringen des Leitungsabschnittes in die Öffnungseinrichtung 10 zum Öffnen des Verschlußelementes kann somit durch einen Mechanismus unterstützt werden, wie er in Figur 2b in Form der federbelasteten Backen 14 gezeigt ist. Das Bezugszeichen 16 kennzeichnet die frontseitigen und das Bezugszeichen 18 die bodenseitige Abdeckung, die zusätzlich als Brecheinrichtung für Standardanschlüsse dient.

Die Öffnungseinrichtung 10 gemäß Figur 1, 2 arbeitet nach dem Zangenprinzip. Sie kann einen gestreckten oder auch einen abgewinkelten Antrieb aufweisen. Die Öffnungseinrichtung 10 ist in ihrer Kontur derart ausgebildet, dass die Verschlußelemente bzw. die diese enthaltenden Leitungsabschnitte nur so eingelegt werden können, dass sie an der vorbestimmten Stelle gedrückt und damit die Leitungen geöffnet werden. Dieses Schlüssel-Schloß-Prinzip verhindert, dass versehentlich Leitungsabschnitte eingelegt werden, in denen kein solches Verschlußelement angeordnet ist. Dies hätte zur Folge, dass zwar die Öffnungseinrichtung betätigt wird, jedoch kein Öffnen der Leitung erfolgt.

Die Öffnungseinrichtungen 10 sind des weiteren mit einer Prozessoreinheit verbunden, die über einen Sensor der Öffnungseinrichtung 10 die Information erhält, dass ein Leitungsabschnitt mit Verschlußelement eingelegt ist und sich in der korrekten Position befindet. Diese Prozessoreinheit überwacht während des Öffnungsvorgangs dessen einwandfreien Verlauf, was beispielsweise mittels Kraftmesselement, Drehmomentenüberwachung oder Motorstrommessung erfolgen kann. Bei falsch eingelegtem oder fehlendem Leitungsabschnitt bzw. Verschlußelement oder bei nicht einwandfrei geöffnetem Verschlußelement kann die Prozessoreinheit den Öffnungsprozess beispielsweise unterbrechen, abbrechen oder ein Hinweissignal bzw. einen Alarm auslösen.

Die erfindungsgemäße Öffnungseinrichtung kann kombiniert sein mit einer Hilfe zum Öffnen von Standardverschlüssen (Bezugszeichen 18 in Fig. 2b), die durch Knicken geöffnet werden. Sie kann so ausgelegt sein, dass die Verwendung herkömmlicher Blutbeutelsysteme nicht behindert, sondern durch die genannte Brechhilfe unterstützt wird.

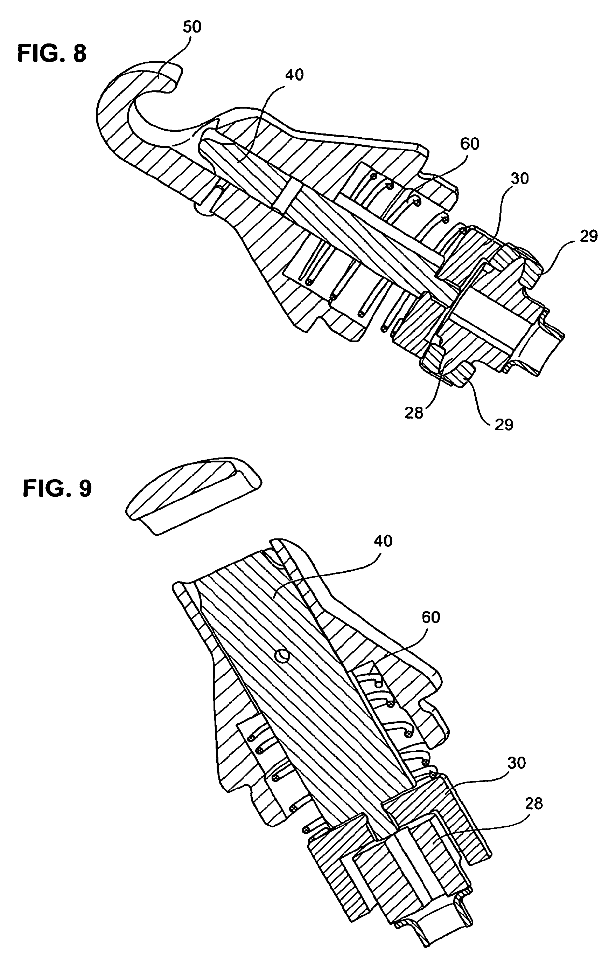

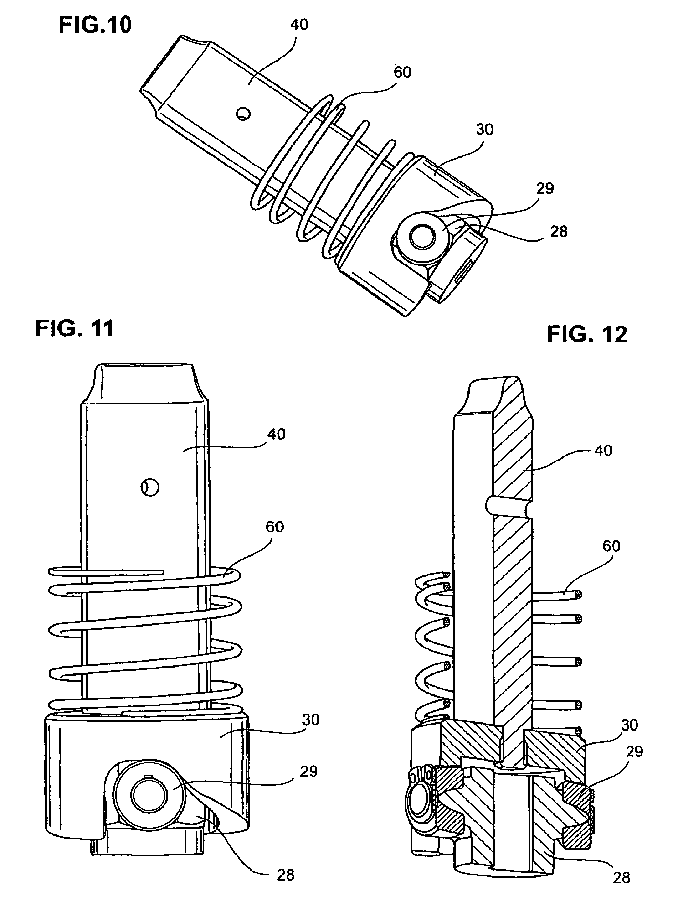

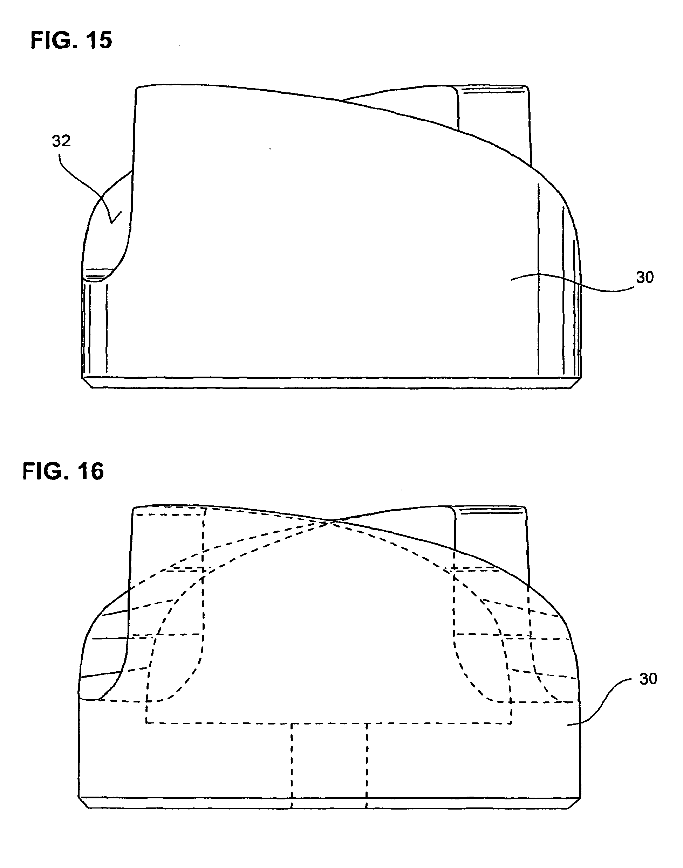

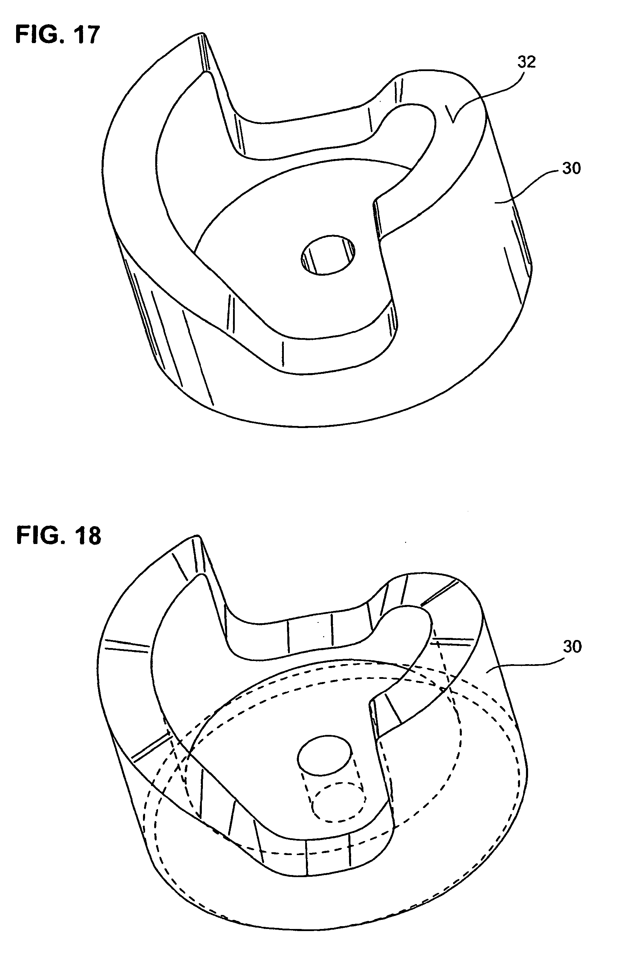

Die Figuren 3 bis 18 betreffen eine handgehaltene Einrichtung 20 zum Öffnen von Verschlußelementen, wie sie aus der DE 10 2005 019 855 A1 bekannt sind.

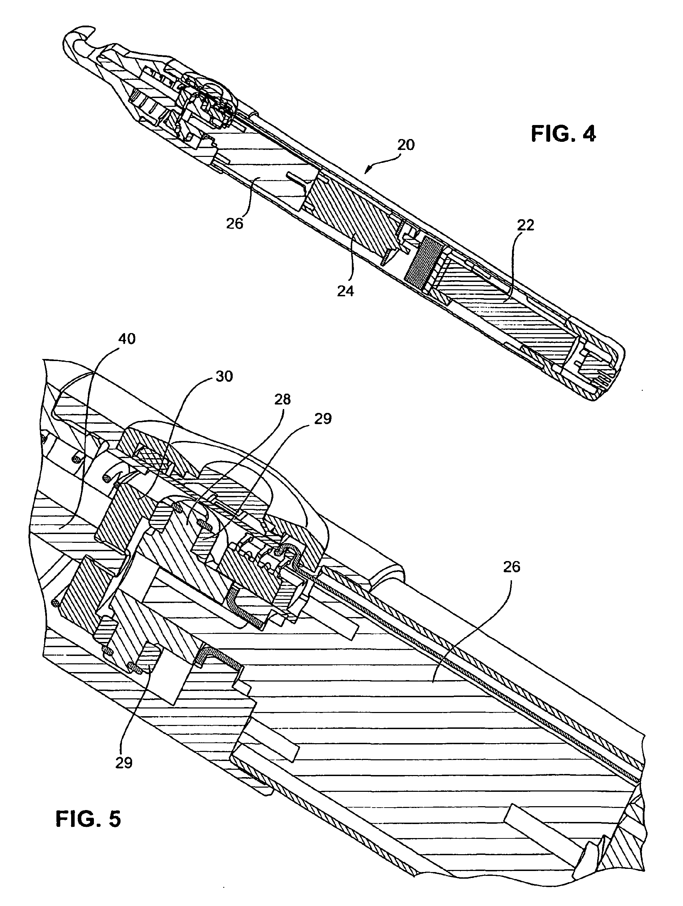

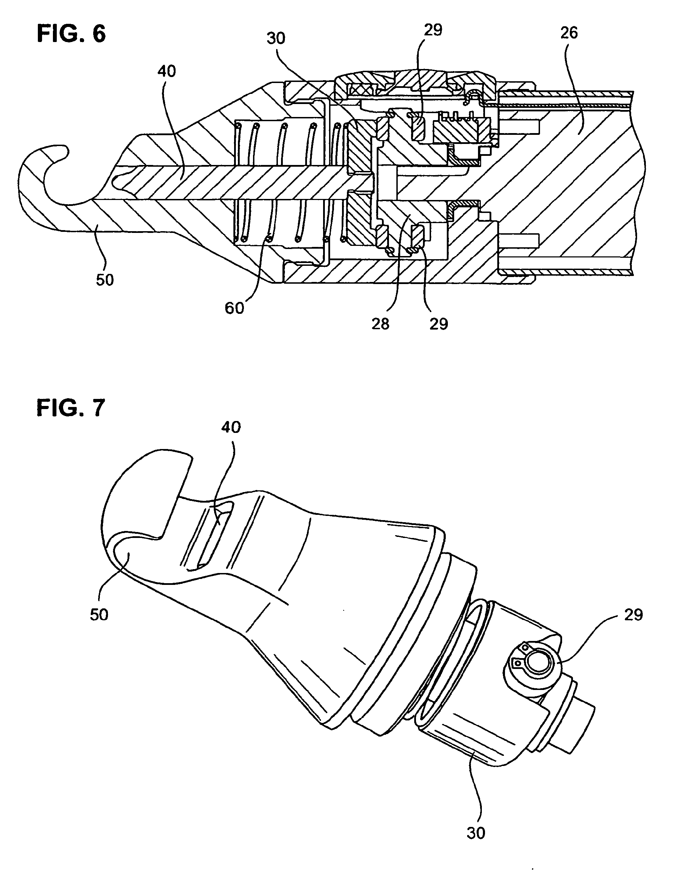

Wie dies aus Figur 3 und 4 hervorgeht, weist die handgehaltene Einrichtung eine stabförmige Gestalt auf. Sie weist einen Akku oder eine Batterie 22 auf, der einen Gleichstrommotor 24 antreibt. Bei Betätigung des Motors 24 wird der Rotationskörper 28 über das mit dem Motor 24 in Verbindung stehende Getriebe 26 angetrieben, d.h. in eine Drehbewegung versetzt. Der Rotationskörper 28 weist in seinen beiden diametral gegenüberliegenden Bereichen Achsen auf, auf denen drehbare Räder 29 angeordnet sind. Dies ergibt sich beispielsweise aus den Detaildarstellungen aus den Figuren 5 und 6. Diese Räder 29 laufen in einer Kulissenführung 30, die ihrerseits drehfest mit einem Öffnungselement 40 in Verbindung steht, das insbesondere aus Figur 6 hervorgeht. Die Figuren 7 bis 12 zeigen die Anordnung der Kulissenführung sowie der durch den Rotationskörper 28 angetriebenen Räder 29 nochmals in verschiedenen perspektivischen sowie Schnittdarstellungen.

Wird der Rotationskörper 28 durch den Motor 24 bzw. über das Getriebe 26 betätigt, rollen die Räder 29 auf der Oberfläche der Kulissenführung 30 ab, was dazu führt, dass es zu einer axialen Ausschub- oder Einfahrbewegung des Öffnungselementes 40 kommt. Die Figuren 7 bis 9 zeigen das Öffnungselement in der eingefahrenen Position. Soll ein Sterilverschluß geöffnet werden, wird der entsprechende Leitungsabschnitt in den hakenförmigen Endbereich 50 der Einrichtung 20 eingelegt und sodann aufgrund einer Tastenbetätigung durch den Nutzer das Öffnungselement 40 ausgefahren, so dass es zu einem Zusammendrücken des Verschlußelementes und somit zu einem Öffnen der Leitung kommt. Die Rückstellbewegung des Öffnungselementes wird durch die Feder 60 bewirkt, die insbesondere aus den Figuren 7 bis 12 hervorgeht. Das Öffnungselement fährt bei seiner Betätigung in diesen hakenförmigen Endabschnitt bzw. in den durch diesen gebildeten Freiraum teilweise ein und drückt so die Leitung zusammen.

Denkbar ist es, dass die Einrichtung gemäß Figur 3, 4 einen Sensor aufweist, der automatisch erkennt, dass die Leitung vorschriftsmäßig eingelegt wurde und automatisch den Öffnungsvorgang initiiert. Ebenfalls ist es denkbar, dass der Benutzer durch eine Benutzerführung dazu veranlaßt wird, durch Betätigung einer Taste oder eines Schalters eine Ausfahrbewegung des Öffnungselementes 40 zu bewirken.

Die Einrichtung gemäß Figur 3, 4 kann beispielsweise über eine Drahtverbindung oder auch schnurlos mit dem Blutbehandlungsgerät in Verbindung stehen und von diesem die entsprechenden Signale erhalten. Die Kommunikation zwischen diesen beiden Einheiten kann auch bidirektional sein und beispielsweise die Übertragung von Daten umfassen, aus denen hervorgeht, dass der Öffnungsvorgang erfolgt ist.

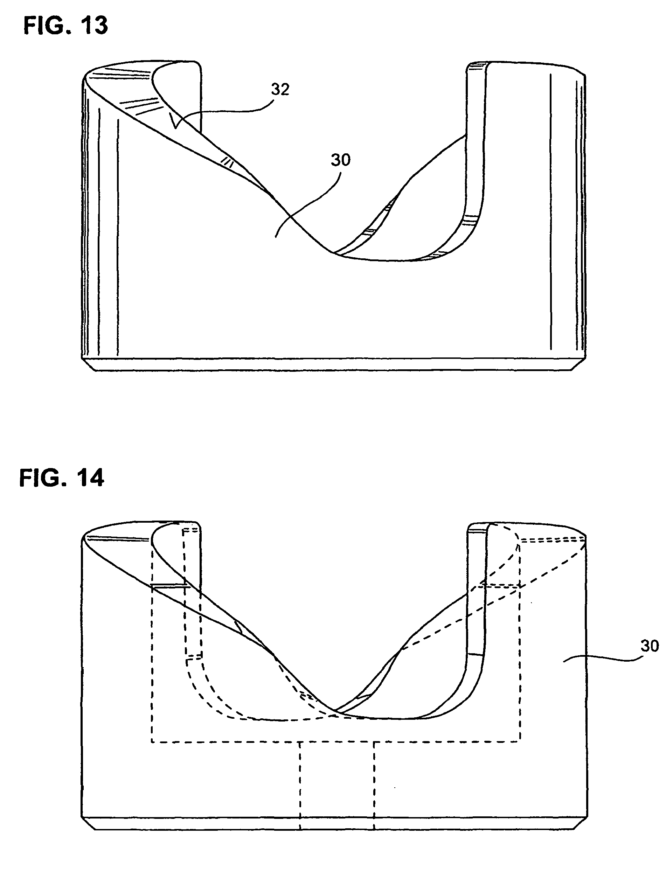

Die Figuren 13 bis 18 zeigen unterschiedliche perspektivische Darstellungen der Kulissenführung 30 sowie von deren Konturlinien. Die Kulissenführung 30 ist im wesentlichen als Hohlzylinderabschnitt ausgeführt und weist in einem Endbereich stirnseitig die Führungsfläche 32 auf, auf der die Rädchen oder Gleitkörper oder dergleichen laufen, die durch die Antriebseinheit in eine Drehbewegung versetzt werden. Die Konturierung der Oberfläche 32 der Kulissenführung 30 ist dabei derart ausgestaltet, dass sie keine konstante Neigung aufweist, sondern dass ein nahezu konstantes Drehmoment erforderlich ist, um die bis auf einen Maximalwert stetig ansteigende Kraft zum Öffnen des Verschlußelementes aufgebracht wird. Eine solche Ausführungsform hat den Vorteil, dass der Antrieb vergleichsweise klein, leicht und billig ausgeführt werden kann.

## Patentansprüche

1. Vorrichtung zum Öffnen einer Leitung, insbesondere der Leitung eines Blutschlauch- oder Blutbeutelsystems, wobei die Leitung ein Schlauch ist, in dem sich ein den Leitungsdurchlass absperrendes Verschlusselement befindet, wobei die Vorrichtung wenigstens eine Öffnungseinrichtung aufweist, die derart ausgeführt ist, dass sie eine von außen auf die Leitung wirkende Kraft ausübt, wodurch das Verschlusselement zusammengepresst und die Leitung geöffnet wird,
**dadurch gekennzeichnet, dass**
die Öffnungseinrichtung derart konturiert ist, dass die Leitung nur derart in die Öffnungseinrichtung einlegbar ist, dass die auf die Leitung wirkende Kraft ausschließlich oder zumindest auch in dem Bereich ausgeübt wird, in dem sich das Verschlusselement befindet.

2. Vorrichtung nach Anspruch 1, **dadurch gekennzeichnet, dass** die Vorrichtung des Weiteren über Mittel verfügt, die ein nur teilweises Einlegen der Leitung in die Öffnungseinrichtung verhindern, wobei die Mittel vorzugsweise über einen Federmechanismus verfügen, der derart ausgestaltet ist, dass die Leitung entweder aus der Öffnungseinrichtung gedrückt wird oder in die korrekte Lage in der Öffnungseinrichtung gedrückt wird.

3. Vorrichtung nach einem der vorhergehenden Ansprüche, **dadurch gekennzeichnet, dass** die Vorrichtung oder die Öffnungseinrichtung wenigstens einen Sensor aufweist, der derart ausgeführt ist, dass er die Anwesenheit der Leitung in der Öffnungseinrichtung und/oder deren Position in der Öffnungseinrichtung und/oder wenigstens einen für den Öffnungsvorgang des Verschlusselementes charakteristischen Parameter erfasst.

4. Vorrichtung nach einem der vorhergehenden Ansprüche, **dadurch gekennzeichnet, dass** die Öffnungseinrichtung Bestandteil einer handgehaltenen Einrichtung ist.

5. Vorrichtung nach Anspruch 4, **dadurch gekennzeichnet, dass** die handgehaltene Vorrichtung über Mittel verfügt, die derart ausgeführt sind, dass sie dem Nutzer der handgehaltenen Vorrichtungen signalisieren, dass ein Öffnungsvorgang eines Verschlusselementes vorzunehmen ist.

6. Vorrichtung nach einem der vorhergehenden Ansprüche, **dadurch gekennzeichnet, dass** es sich bei der Vorrichtung um ein Blutbehandlungsgerät oder einen Bestandteil eines Blutbehandlungsgerät handelt.

7. Verwendung einer Vorrichtung nach einem der vorhergehenden Ansprüche zum Öffnen eines Schlauches, insbesondere eines Schlauches eines Blutschlauch- oder Blutbeutelsystems, in dem sich ein Verschlusselement befindet.

## Claims

1. Device for opening a line, in particular the line of a blood tube system or blood bag system, wherein the line is a tube in which a sealing element is located that blocks the passage through the line, wherein the device has at least one opening mechanism, which is designed in such a way that it exerts a force that acts on the line from the outside, as a result of which the sealing element is compressed and the line is opened, **characterized in that** the opening mechanism is contoured in such a way that the line can be introduced into the opening mechanism only in such a way that the force acting on the line is exerted exclusively, or at least also, in the area in which the sealing element is located.

2. Device according to Claim 1, **characterized in that** the device further comprises means that prevent an only partial introduction of the line into the opening mechanism, wherein the means preferably comprise a spring mechanism, which is designed in such a way that the line is either pressed out of the opening mechanism or pressed into the correct position in the opening mechanism.

3. Device according to one of the preceding claims, **characterized in that** the device or the opening mechanism has at least one sensor, which is designed in such a way that it detects the presence of the line in the opening mechanism and/or the position of the line in the opening mechanism and/or at least one parameter characteristic for the opening procedure of the sealing element.

4. Device according to one of the preceding claims, **characterized in that** the opening mechanism is a component of a hand-held mechanism.

5. Device according to Claim 4, **characterized in that** the hand-held device has means which are designed in such a way that they signal to the user of the hand-held devices that an opening procedure of a sealing element should be carried out.

6. Device according to one of the preceding claims, **characterized in that** the device is a blood treatment appliance or a component of a blood treatment appliance.

7. Use of a device according to one of the preceding claims for opening a tube, in particular a tube of a blood tube system or blood bag system, in which a sealing element is located.

## Revendications

1. Dispositif pour ouvrir une ligne, en particulier la ligne d'un système de tube sanguin ou de poche de sang, la ligne étant un tube dans lequel se trouve un élément de fermeture bloquant le passage à travers la ligne, le dispositif présentant au moins un système d'ouverture qui est réalisé de telle sorte qu'il exerce depuis l'extérieur une force agissant sur la ligne de sorte que l'élément de fermeture soit comprimé et que la ligne soit ouverte,
**caractérisé en ce que**
le système d'ouverture est profilé de telle sorte que la ligne ne puisse être introduite dans le système d'ouverture que de telle sorte que la force agissant sur la ligne soit exercée exclusivement ou au moins aussi dans la région dans laquelle se trouve l'élément de fermeture.

2. Dispositif selon la revendication 1, **caractérisé en ce que** le dispositif dispose en outre de moyens qui empêchent une introduction seulement partielle de la ligne dans le système d'ouverture, les moyens disposant de préférence d'un mécanisme à ressort qui est configuré de telle sorte que la ligne soit pressée hors du système d'ouverture ou qu'elle soit pressée dans la position correcte dans le système d'ouverture.

3. Dispositif selon l'une quelconque des revendications précédentes, **caractérisé en ce que** le dispositif ou le système d'ouverture présente au moins un capteur qui est réalisé de telle sorte qu'il détecte la présence de la ligne dans le système d'ouverture et/ou sa position dans le système d'ouverture et/ou au moins un paramètre caractéristique de l'opération d'ouverture de l'élément de fermeture.

4. Dispositif selon l'une quelconque des revendications précédentes, **caractérisé en ce que** le système d'ouverture fait partie d'un dispositif tenu à la main.

5. Dispositif selon la revendication 4, **caractérisé en ce que** le dispositif tenu à la main dispose de moyens qui sont réalisés de telle sorte qu'ils signalent à l'utilisateur des dispositifs tenus à la main qu'une opération d'ouverture d'un élément de fermeture doit être effectuée.

6. Dispositif selon l'une quelconque des revendications précédentes, **caractérisé en ce que** le dispositif est un appareil de traitement sanguin ou un constituant d'un appareil de traitement sanguin.

7. Utilisation d'un dispositif selon l'une quelconque des revendications précédentes pour ouvrir un tube, en particulier un tube d'un système de tube sanguin ou de poche de sang, dans lequel se trouve un élément de fermeture.
